# FASCICULE DE BREVET EUROPEEN

(11) **EP 2 274 027 B1**
(45) Date de publication et mention de la délivrance du brevet: **18.01.2017**
(21) Numéro de dépôt: 09730853.0
(22) Date de dépôt: 08.04.2009
(51) Int. Cl.: A61M 5/178, A61M 5/315, A61M 5/50, A61M 5/24

(54) **PROCEDE DE FABRICATION D'UN DISPOSITIF D'EJECTION JETABLE**
VERFAHREN ZUR HERSTELLUNG EINER ENWEG-INJEKTIONSVORRICHTUNG
METHOD OF MANUFACTURING A DISPOSABLE EJECTION DEVICE

(30) Priorité: 10.04.2008 FR 0801972
(43) Date de publication de la demande: 19.01.2011
(73) Titulaire: Primequal S.A., 1268 Begnins (CH)
(72) Inventeur: WEILL, David, CH-1268 Begnins (CH); CHASSOT, Pierre-Yves, F-01710 Thoiry (FR)
(74) Mandataire: Bugnion Genève
(86) Numéro de dépôt international: PCT/IB2009/051475
(87) Numéro de publication internationale: WO 2009/125353

(56) Documents cités:
- EP-A- 1 263 387
- WO-A-03/082387
- WO-A-2005/007224
- FR-A- 2 535 206
- US-A- 4 444 560
- US-A1- 2001 049 506
- US-A1- 2002 056 486
- US-A1- 2003 105 430

## Description

L'invention concerne un procédé de fabrication d'un dispositif d'éjection d'un produit liquide ou pâteux. Elle concerne aussi un kit de fabrication d'un tel dispositif.

Il est connu de la demande de brevet FR 2 535 206 une seringue dentaire pour injection intra-ligamentaire. Cette seringue permet l'injection par une aiguille très fine et souple d'un produit dans les ligaments situés entre l'os de la mâchoire et la dent. Elle est principalement constituée d'un corps allongé sur lequel est monté un mécanisme commandant l'injection par le biais du déplacement d'un cylindre de poussée, d'un porte-conteneur dans lequel est logé un conteneur rempli de liquide à injecter et d'un embout comportant l'aiguille d'injection. Afin de résoudre des problèmes d'accès difficile aux zones où doivent être faites les injections, le corps de la seringue présente une tête d'injection qui fait un angle avec l'axe du corps de la seringue. L'aiguille, amovible, est mise en place sur le corps avant de pratiquer les injections puis retirée après. Le mécanisme commandant l'injection est composé principalement d'un levier articulé sur le corps de seringue et agissant sur un cylindre de poussée par le biais d'un cliquet articulé sur le levier et rappeler dans une position de contact avec les dents d'une crémaillère réalisées sur le cylindre de poussée. Le cylindre est guidé en translation dans un alésage réalisé dans le corps de seringue. Il présente en outre une rainure longitudinale coopérant avec une vis vissée radialement par rapport à l'alésage et débouchant dans celui-ci pour interdire la rotation du cylindre. Le mécanisme présente de plus un cliquet anti-retour interdisant le recul du cylindre de poussée lorsqu'on met fin à l'action sur le levier. Ce cliquet anti-retour est rappelé dans une position de contact avec les dents de la crémaillère et peut être éloigné de cette position par action sur un bouton pour annuler la pression d'injection et/ou pour changer le conteneur de produit à injecter sur lequel appuie le cylindre de poussée. Un tel dispositif présente des inconvénients. D'une part, sa réalisation est complexe et coûteuse. D'autre part, il présente de nombreuses pièces et des formes complexes, en particulier, des angles et des coins dans la matière. Ces angles et coins forment des zones très peu accessibles et, par conséquent, très difficiles à nettoyer et donc difficiles à stériliser.

Pour pallier aux inconvénients précédents, le document WO2005/007224 propose un dispositif d'éjection présentant une construction plus simple, aisément démontable et nettoyable. Ce dispositif comprend un corps, une partie destinée à contenir le produit et munie d'un orifice pour l'éjection du produit, un cylindre de poussée muni de dents, se déplaçant dans un alésage du corps et faisant varier le volume de la partie destinée à contenir le produit et un mécanisme de déplacement du cylindre de poussée lié au corps, comprenant un levier articulé démontable, rappelé par un ressort de rappel, agissant sur les dents du cylindre de poussée par le biais d'un cliquet articulé sur le levier et rappelé dans une position de contact avec le cylindre de poussée par un ressort et un cliquet anti-retour rappelé dans une position de contact avec le cylindre de poussée. Malgré sa plus grande simplicité et la présence d'un levier démontable facilitant le nettoyage, ce dispositif n'atteint toujours pas un degré d'hygiène satisfaisant et nécessite toujours une phase de nettoyage délicate. De plus, la liaison démontable du levier avec le corps du dispositif est obtenue au détriment de l'efficacité du dispositif, car elle induit une moins bonne tenue du levier qui subit des efforts importants lors de la réalisation d'une éjection. Enfin, cette solution comme la précédente nécessite au moins un pré-assemblage partiel avant sa distribution, sinon son assemblage se révèlerait trop contraignant du fait du nombre d'éléments du dispositif.

Le US 2002/056486A1 et le WO 01/60311A1 décrivent des procédés de fabrication de dispositifs d'éjection comprenant, notamment, une étape de remplissage d'un porte-conteneur par refoulement du produit à éjecter.

Toutefois, ces procédés ne peuvent être mis en oeuvre de façon satisfaisante pour réaliser des dispositifs d'éjection prêts à l'emploi et intégrant des porte-conteneurs de différentes capacités dans lesquels le corps arrière assurant l'actionnement du piston interne pour l'éjection du produit est assemblé de manière irréversible au porte-conteneur.

Finalement, toutes les solutions existantes reposent sur des dispositifs complexes dont le procédé de fabrication est coûteux et inadapté. De plus, elles sont insatisfaisantes en terme d'hygiène.

Ainsi, un objet général de l'invention est de proposer un procédé de fabrication d'un dispositif d'éjection d'un produit liquide ou pâteux palliant aux inconvénients des solutions existantes.

Plus précisément, un premier objet est de proposer un procédé de fabrication économique d'un dispositif d'éjection.

Un second objet est de proposer un procédé de fabrication d'un dispositif d'éjection d'un produit liquide ou pâteux offrant un degré d'hygiène maximal.

A cet effet, l'invention repose sur un procédé de fabrication d'un dispositif d'éjection d'un produit liquide ou pâteux caractérisé en ce qu'il comprend :
- au moins trois étapes de moulage par injection d'un matériau plastique pour former trois parties distinctes du dispositif ; un levier intégrant un cliquet, un porte conteneur et un corps arrière,
- une étape par laquelle une tirette est insérée dans une ouverture arrière du porte conteneur et reliée au piston délimitant le volume à injecter,
- une étape d'aspiration du produit à éjecter au sein du volume du porte conteneur du dispositif d'éjection à l'aide de la tirette
- une étape de démontage de la tirette du porte conteneur,
- une étape intermédiaire d'assemblage du levier sur le corps arrière, suivie de l'insertion d'une crémaillère par une ouverture arrière, et
- une étape d'assemblage irréversible du corps arrière sur le porte conteneur pour former le dispositif d'éjection prêt à l'emploi.

Le procédé peut comprendre une étape préalable d'assemblage de la tirette directement ou indirectement avec un piston du porte conteneur du dispositif d'éjection fermant le volume à remplir. Pour cela, la liaison entre la tirette et le piston du porte conteneur peut se faire par un vissage de l'extrémité de la tirette dans le piston.

De plus, le procédé de fabrication peut comprendre une étape préalable de fixation d'un outil à l'extrémité avant du porte conteneur. Pour cela, l'extrémité antérieure du porte conteneur peut présenter une forme globalement inclinée par rapport au corps mais présentant une surface ayant au moins une génératrice sensiblement parallèle à l'axe de ce corps afin de permettre une insertion droite d'un outil de type aiguille dans cette direction avant sa flexion et fixation en position de travail inclinée.

En variante, le procédé de fabrication peut comprendre une étape préalable d'assemblage avec le porte conteneur d'un dispositif de type seringue présentant un corps cylindrique allongé délimité dans sa partie arrière par un piston mobile en translation longitudinale, et comprenant dans sa partie avant une aiguille maintenue par un embout.

De plus, le procédé peut comprendre une étape intermédiaire d'assemblage d'un levier sur le corps arrière. Pour cela, le levier peut comprendre une partie supérieure horizontale s'étendant vers l'avant, destinée à être manipulée, et une extrémité inférieure en forme d'axe qui est positionnée dans un emplacement correspondant du corps arrière. En variante, le procédé de fabrication peut comprendre une étape intermédiaire d'assemblage d'une crémaillère avec le corps arrière par son insertion par une ouverture.

L'étape d'assemblage du corps arrière avec le porte conteneur peut être réalisée par clipsage d'au moins deux lames latérales d'un premier élément, s'étendant longitudinalement et présentant une protubérance orientée vers l'extérieur, destinées à coopérer avec des ouvertures correspondantes sur le second élément. Pour cela, l'étape d'assemblage du corps arrière avec le porte conteneur est irréversible.

Ce procédé peut de plus comprendre l'assemblage d'une crémaillère au sein du dispositif, cette crémaillère étant apte à un mouvement dans une certaine direction pour induire l'éjection du produit et dont le mouvement dans la direction opposée est impossible de sorte d'empêcher un second remplissage du dispositif.

Le procédé de fabrication d'un dispositif d'éjection peut comprendre au moins trois étapes d'injection de matériau plastique pour former trois parties distinctes du dispositif, un levier intégrant un cliquet, un porte conteneur et un corps arrière.

En outre, le procédé de fabrication peut comprendre une quatrième étape d'injection en matériau plastique pour former une crémaillère.

L'assemblage du corps arrière sur le porte conteneur peut comprendre une liaison entre un moyen d'éjection du corps arrière et un piston du porte conteneur de sorte de pouvoir éjecter le produit du porte conteneur par une action sur ce moyen d'éjection du corps arrière.

L'invention porte aussi sur un kit de fabrication, soit un ensemble d'éléments prévus pour la fabrication, d'un dispositif d'éjection d'un produit liquide ou pâteux, caractérisé en ce qu'il comprend un porte conteneur, un corps arrière et une tirette.

Pour cela, la tirette peut être distincte du porte conteneur et présenter un organe de liaison pour sa liaison directe ou indirecte avec un piston du porte conteneur.

Le kit de fabrication peut de plus comprendre un levier comprenant un cliquet, et une crémaillère, destinés à une liaison avec le corps arrière.

Le kit de fabrication peut de plus comprendre un porte outil et un outil de type aiguille, aptes à une liaison avec le porte conteneur.

Ces objets, caractéristiques et avantages, ainsi que d'autres de la présente invention seront exposés en détail dans la description suivante d'un mode d'exécution particulier fait à titre non-limitatif en relation avec les figures jointes parmi lesquelles :
Les figures 1 à 3 représentent schématiquement des étapes d'assemblage d'un procédé de fabrication d'un dispositif d'éjection selon un mode de réalisation de l'invention ;
La figure 4 représente le dispositif d'éjection obtenu par le procédé de fabrication selon le mode d'exécution de l'invention ;
les figures 5a et 5b représentent un détail de ces étapes d'assemblage selon un mode de réalisation de l'invention ;
la figure 6 illustre une étape préalable du procédé de fabrication selon un mode d'exécution de l'invention ;
les figures 7a et 7b illustrent une variante d'exécution de l'étape préalable précédente selon un mode d'exécution de l'invention ;
la figure 8 illustre une étape intermédiaire selon un mode d'exécution de l'invention ;
la figure 9 représente une vue en perspective d'un assemblage intermédiaire du levier du dispositif d'éjection ;
la figure 10 représente une vue en coupe de l'assemblage intermédiaire du levier du dispositif d'éjection ;
la figure 11 représente une vue en perspective de la dernière étape d'assemblage du dispositif d'éjection selon un mode de réalisation de l'invention ;
les figures 12 et 13 représentent des vues partielles en perspective de cette dernière étape afin d'illustrer des éléments complémentaires ;
la figure 14 représente une vue du dispositif d'éjection selon le mode de réalisation de l'invention en perspective avant coupée selon un plan vertical longitudinal passant approximativement au milieu du dispositif.

Selon le mode d'exécution préféré de l'invention, le procédé de fabrication du dispositif d'éjection repose sur l'assemblage de quatre principaux éléments distincts, de forme simple permettant leur fabrication en matériau plastique par simple injection.

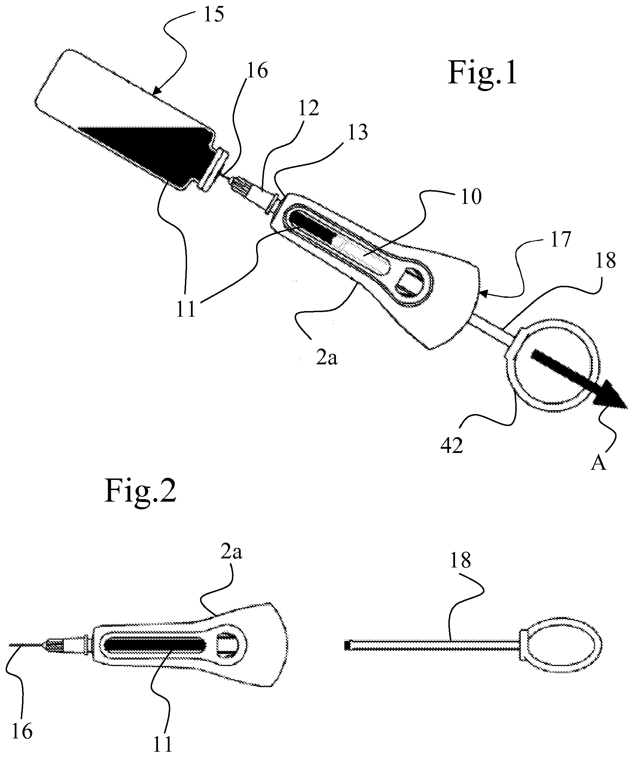

Le procédé de fabrication de ce dispositif d'éjection comprend les trois étapes essentielles suivantes, illustrées sur les figures 1 à 3.

Dans une première étape illustrée sur la figure 1, le volume 10 du porte conteneur 2a du dispositif d'éjection est rempli du produit 11 à éjecter par un principe d'aspiration depuis un flacon 15 de stockage. Pour cela, le porte conteneur 2a est équipé à son extrémité avant 13 d'un porte-outil 12 permettant de fixer une aiguille 16. Vers son extrémité arrière, le porte conteneur 2a comprend une ouverture 17 par laquelle une tirette 18 sous forme de tige a été inséré et relié au piston 19 délimitant le volume 10 portant le contenu à éjecter, mobile dans la direction longitudinale selon la longueur du porte conteneur 2a. Par la traction de la tirette 18, l'utilisateur entraîne le piston 19, qui se trouve en position avancée en début de cette étape, vers l'arrière selon la direction A, ce qui entraîne l'aspiration du produit 11 depuis le flacon 15 vers l'intérieur du volume 10 du porte conteneur 2a. En remarque, la tirette 18 est munie d'un anneau de préhension 42 dans sa partie arrière pour faciliter sa manipulation.

Dans une seconde étape illustrée par la figure 2, qui est engagée lorsque le porte conteneur 2a contient la quantité de produit 11 souhaitée, la tirette 18 est désolidarisé du piston 19. Cette tirette est un composant qui n'appartient pas au dispositif d'éjection mais intervient seulement dans cette phase intermédiaire pour permettre un remplissage simple et avantageux du dispositif d'éjection.

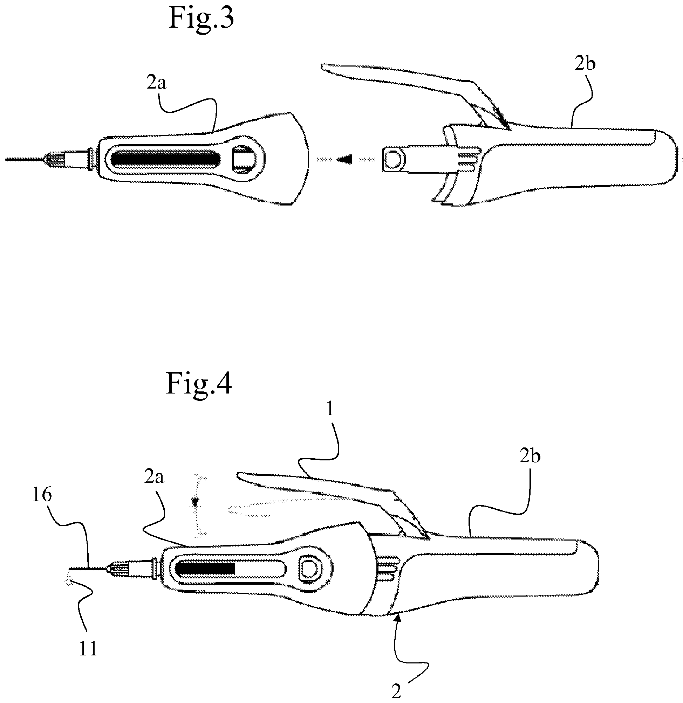

Dans une troisième étape illustrée par la figure 3, le dispositif d'éjection est définitivement assemblé par le montage d'un corps arrière 2b sur le porte conteneur 2a, ce corps arrière remplissant la fonction mécanique d'éjection du produit 11 contenu dans le porte conteneur 2a. Pour cela, il comprend des moyens d'éjection qui vont agir sur le piston 19 du porte conteneur 2a comme cela sera détaillé par la suite. A la fin de cette troisième étape, le dispositif d'éjection, tel qu'illustré par la figure 4, est prêt à l'emploi. Les deux parties 2a et 2b assemblées forment un seul corps 2. Un actionnement du levier 1 permet l'éjection du produit 11 du porte conteneur 2a par l'extrémité de l'aiguille 16.

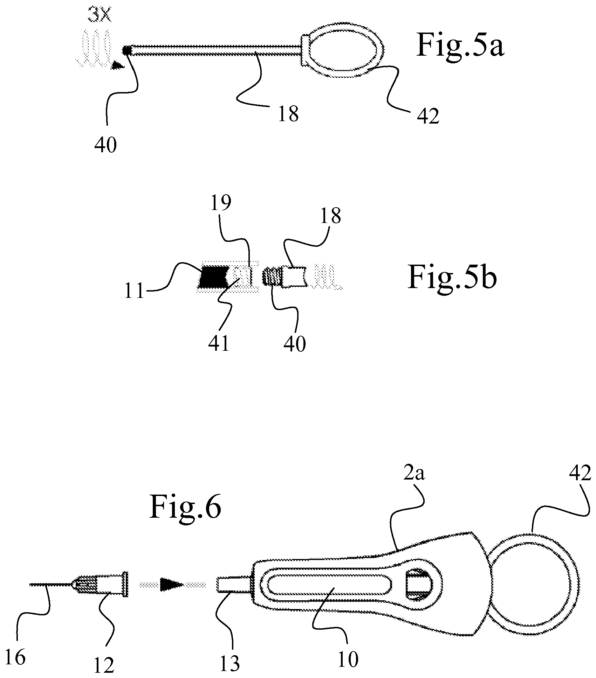

Les figures 5a et 5b montrent la liaison entre la tirette 18 et le piston 19 du porte conteneur 2a, qui est obtenue par un vissage, l'extrémité avant de la tirette comprenant une partie filetée 40 coopérant avec une partie filetée 41 aménagée dans le piston 19. Selon ce mode d'exécution, un simple vissage ou dévissage de trois tours permet respectivement la liaison et la séparation de la tirette et du piston. Toute autre variante de liaison directe ou indirecte entre ces deux éléments est toutefois possible. Ainsi, la première étape décrite ci-dessus en relation avec la figure 1 sera précédée d'une étape de montage de la tirette 18 sur le piston 19, selon une étape opposée à l'étape 2 du procédé de démontage de la tirette.

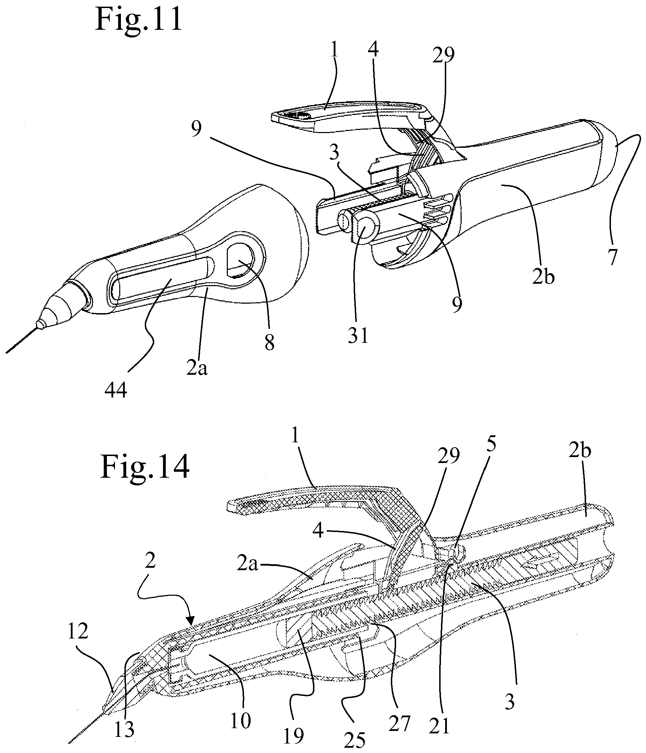

La figure 6 illustre une étape préalable selon un mode d'exécution de l'invention qui consiste en la fixation d'une aiguille 16 à l'extrémité avant 13 du porte conteneur, par l'intermédiaire d'un porte outil 12 qui est ici un porte aiguille. Cette fixation peut se faire par vissage, ou tout autre raccord rapide. Selon une variante avantageuse, cette liaison peut se faire selon l'enseignement du document WO03/082387. Pour cela, la forme antérieure 13 du porte conteneur 2a présente une forme globalement inclinée par rapport au corps 2 mais présentant une surface ayant au moins une génératrice sensiblement parallèle à l'axe de ce corps 2a afin de permettre une insertion droite de l'aiguille 16 dans cette direction avant sa flexion et fixation inclinée en position de travail, tel qu'illustré par la variante de la figure 11.

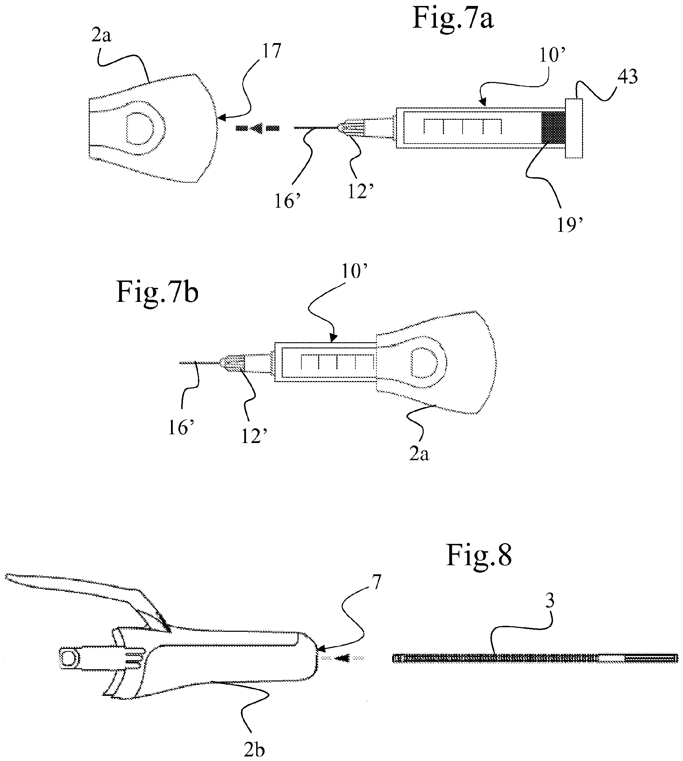

Les figures 7a et 7b illustrent une variante d'exécution de l'étape préalable précédente, qui consiste en l'intégration d'un dispositif de type seringue 10' comprenant une aiguille 16' maintenue par un embout 12' sur un corps cylindrique allongé délimité dans sa partie arrière par un piston 19' mobile en translation longitudinale. Cette seringue 10' est insérée dans le porte conteneur 2a, par son ouverture arrière 17, jusqu'à ce que sa base arrière 43 formant un épaulement vienne en butée dans son emplacement définitif. Cette variante permet aussi d'obtenir un ensemble porte conteneur semblable à la configuration illustrée par la figure 1. Ainsi, le volume 10' du porte conteneur ainsi que le piston 19' peuvent être des composants indépendants assemblés au porte conteneur ou des éléments obtenus par la structure même du porte conteneur.

La variante précédente illustre ainsi que l'extrémité 13 du porte conteneur et l'embout 12 pour la fixation de l'outil peuvent être un seul et même élément. Une telle configuration sera par exemple avantageusement implémentée dans le cadre d'un dispositif d'éjection d'une colle.

La figure 8 illustre une étape intermédiaire du procédé d'assemblage, précédant l'ultime étape 3, qui consiste en un assemblage d'une crémaillère 3 avec le corps arrière 2b par son insertion par une ouverture arrière 7 réalisée dans ce corps arrière, jusqu'à sa position dans laquelle sa face avant est apte à une coopération avec le piston 19 du porte conteneur.

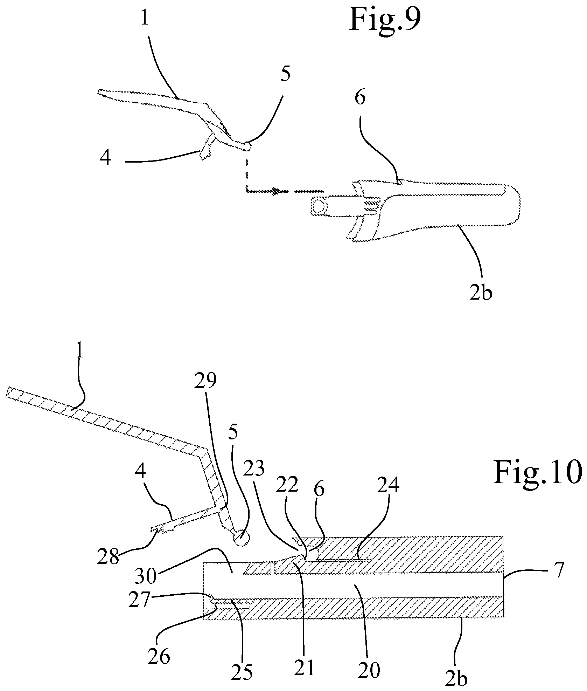

Les figures 9 et 10 illustrent une autre étape intermédiaire du procédé d'assemblage, précédant l'ultime étape 3, qui consiste en l'assemblage d'un levier 1 avec le corps arrière 2b. Le levier 1 comprend une partie supérieure horizontale s'étendant vers l'avant, destinée à être manipulée, et une extrémité inférieure en forme d'axe 5 qui est positionnée dans un emplacement 6 correspondant du corps arrière 2b. Un cliquet 4 est de plus lié au levier 1 selon une structure monolithique et s'étend vers le bas, vers l'intérieur du corps 2b.

La figure 10 représente plus particulièrement la liaison entre le levier 1 et le corps arrière 2b et permet d'observer en détail les éléments mis en oeuvre lors de cette phase intermédiaire. Le levier 1 comprend une extrémité 5 cylindrique, formant son axe de rotation, qui vient se loger dans un emplacement cylindrique 6 de diamètre correspondant formé dans la partie supérieure du corps arrière 2b du dispositif. Cet emplacement cylindrique 6 comprend dans sa partie inférieure une lame élastique longitudinale 21 intégrée au corps 2b, formée par une découpe 24 d'une partie de la surface supérieure de l'alésage cylindrique central 20 du corps arrière 2b. L'axe 5 est d'abord positionné sur deux rails latéraux non représentés réalisés dans la partie supérieure du corps 2, puis déplacé vers l'arrière en glissant sur ces rails le conduisant vers l'ouverture 23 de l'emplacement 6 final, ouverture de plus petite dimension que le diamètre de l'axe 5, la lame 21 s'effaçant vers le bas au sein de l'alésage 20 sous la pression de l'axe 5 du levier 1 pour le laisser passer et atteindre son emplacement 6 par cette ouverture 23. La lame 21 reprend ensuite sa position naturelle grâce à son élasticité et vient épouser une partie inférieure de la circonférence de l'axe 5.

Le levier 1 est de plus relié à un cliquet 4 sensiblement perpendiculaire au levier en une zone de liaison 29. Ce cliquet 4 se termine par une extrémité 28 comprenant une ou plusieurs dents pour coopérer avec la crémaillère 3, non encore reliée au corps 2b. Le cliquet 4 et le reste du levier 1 forment une structure monolithique, obtenue par une seule étape d'injection. Leur zone de liaison 29 est telle que le cliquet 4 est mobile élastiquement par rapport au levier 1, selon une rotation autour de sa liaison 29 avec le levier. Pour cela, des découpes ou des zones de moindre épaisseur sont prévues au sein du matériau plastique dans la zone de liaison 29, de sorte de former une zone de moindre rigidité, déformable par rapport au reste du levier. Ces découpes peuvent par exemple dessiner un axe s'étendant sur la largeur du levier dans la zone 29, et relié au cliquet 4 pour représenter son axe de rotation.

Enfin, le corps arrière 2b présente au moins un cliquet anti-retour 25, sous la forme d'une lame élastique longitudinale obtenue par une découpe 26 dans le corps lui-même et formée ainsi lors de l'injection du corps 2b, et présentant une extrémité 27 en forme de pointe complémentaire des dents de la crémaillère 3, s'étendant au sein de l'alésage 20, pour coopérer avec ces dents comme cela sera détaillé par la suite.

Lorsque le levier est en position dans l'emplacement 6 du corps 2b, une crémaillère 3 est insérée dans l'alésage cylindrique longitudinal 20 du corps ainsi assemblé par une ouverture arrière 7, comme illustré à la figure 8. Cette insertion de la crémaillère 3 dans l'alésage 20 du corps 2 la conduit en appui sur la surface inférieure de la lame 21 d'insertion du levier 1, empêchant ensuite tout mouvement vers le bas de cette lame 21. Par ce biais, l'axe 5 du levier 1 se trouve verrouillé dans l'emplacement 6 du corps 2, ne pouvant plus s'échapper par l'ouverture 23 de plus petite dimension.

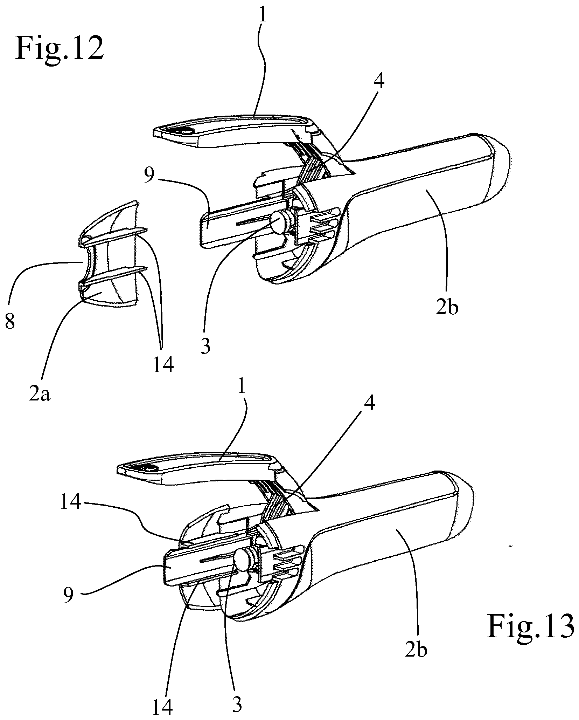

Les figures 11 à 13 illustrent plus précisément l'étape 3 d'assemblage final du corps arrière 2b comprenant la mécanique d'éjection sur le porte conteneur 2a selon le mode d'exécution préféré de l'invention. Pour réaliser cet assemblage, le corps arrière 2b présente deux lames latérales 9 s'étendant longitudinalement vers l'avant, aptes à coopérer avec des rails complémentaires 14 inférieur et supérieur prévus contre les parois latérales à l'intérieur du porte-conteneur 2a. L'extrémité de ces lames 9 présente une protubérance 31 orientée vers l'extérieur, destinée à coopérer avec des ouvertures correspondantes 8 du porte-conteneur 2a. Lors de l'insertion des lames 9 dans les rails 14 du porte-conteneur 2a, les protubérances 31 viennent frotter contre les parois latérales intérieures du porte-conteneur 2a, en provoquant une déformation élastique des lames 9 vers l'intérieur du corps 2. Dès que les protubérances 31 atteignent les ouvertures 8 de forme correspondante, elles y prennent place sous l'effet élastique des lames 9 qui retrouvent leur écartement normal initial et leur direction longitudinale et parallèle, selon un principe de clipsage. La forme allongée des lames 9 maintenues sur leur longueur par les rails 14 inférieur et supérieur permet une liaison rigide et sans jeu entre les deux parties 2a et 2b du corps pour supporter ainsi efficacement les efforts subis lors de son utilisation. Toute autre liaison mécanique équivalente entre ces deux parties pourrait convenir. Notamment, en variante, les lames 9 pourraient se trouver sur le porte conteneur 2a et les ouvertures correspondantes 8 sur le corps arrière. De plus, cette liaison pourrait être de type crochet/taquet, obtenue par un mouvement de rotation des deux parties, par clippage, par une seule lame ou crochet...

Selon une réalisation avantageuse du dispositif, le volume 10 du porte conteneur 2a vient jusqu'aux abords des lames 9 au niveau des ouvertures 8 de sorte que le mouvement inverse du corps arrière 2b, qui nécessiterait une nouvelle déformation des lames 9 en appuyant sur les protubérances 31 pour les extraire des ouvertures 8 tout en tirant le corps 2b vers l'arrière, est rendu difficile, voire impossible. Cette géométrie gêne ainsi cet échappement des protubérances 31 et permet un verrouillage ou quasi-verrouillage du corps 2 ainsi assemblé, qui n'est donc pas ou très difficilement démontable, c'est-à-dire que l'assemblage est irréversible ou quasi-irréversible.

La figure 14 illustre en coupe le dispositif assemblé selon une variante du mode d'exécution. L'extrémité dentée 28 du cliquet 4 du dispositif d'éjection, particulièrement visible sur la figure 10, pénètre dans une ouverture supérieure du corps 2 pour venir dans une zone proche des dents de la crémaillère 3, et la partie extrême 27 du cliquet anti-retour 25 vient se loger dans une dent de la crémaillère. L'insertion de la crémaillère 3 dans l'alésage 20 s'accompagne d'un clic sonore lorsque l'extrémité 27 du cliquet anti-retour 25 prend position entre deux dents de la crémaillère. Comme cette extrémité 27 du cliquet anti-retour 25 occupe une position légèrement plus avancée que celle de l'extrémité 28 du cliquet 4, ce clic sonore intervient quand la crémaillère est suffisamment avancée pour pouvoir être actionnée par le levier 1 et ce clic permet d'informer l'utilisateur que la crémaillère est suffisamment avancée.

Le levier 1 est pressé vers le bas contre le porte-conteneur 2a par une rotation autour de l'axe 5, entraînant une déformation élastique du cliquet 4 en appui sur la crémaillère 3 autour de sa zone de liaison 29 avec le levier, grâce à la propriété de flexibilité du matériau utilisé et la géométrie choisie au niveau notamment de la liaison 29 entre le cliquet 4 et le levier 1. Ce mouvement du cliquet 4 permet à son extrémité dentée 28 qui coopère avec des dents de la crémaillère 3, d'induire une force de poussée sur la crémaillère et son avancée au sein de l'alésage 20, pour agir sur le piston 19 et provoquer l'éjection du produit 11 contenu dans le dispositif.

Lorsque le levier est relâché, le cliquet 4 exerce un effort de rappel élastique vers sa position initiale et entraîne le levier 1 vers sa position haute initiale. Dans cette phase, le cliquet anti-retour 25 empêche la crémaillère de revenir en arrière.

Ce mode d'exécution garantit le caractère jetable du dispositif, qui ne peut être utilisé qu'une seule fois pour éjecter le volume de produit choisi. Cette garantie est obtenue par la combinaison de deux principaux facteurs :
- la liaison entre les deux parties 2a, 2b du corps est irréversible ou quasi-irréversible ;
- lorsque le piston est en bout de course après éjection du produit, il reste verrouillé dans cette position par la crémaillère dont le mouvement arrière est impossible du fait du cliquet anti-retour. Il n'est donc pas possible de remplir de nouveau le volume du porte conteneur.

Comme cela a été exposé précédemment, un procédé de fabrication rapide et peu coûteux du dispositif d'éjection selon un mode de réalisation préféré consiste à fabriquer séparément par injection de matériau plastique les quatre différents composants essentiels du dispositif, le levier 1, le porte-conteneur 2a, le corps arrière 2b et la crémaillère 3. Le matériau utilisé peut être du polyamide, du polypropylène, de l'ABS ou tout autre matériau plastique. Un plastique recyclable sera bien adapté au dispositif qui est jetable après utilisation. Un matériau plastique transparent peut aussi être avantageusement utilisé, notamment pour le porte-conteneur 2a, pour permettre de visualiser le volume restant de produit à éjecter, au moins au travers une fenêtre 44. En variante, les composants précédents peuvent être fabriqués différemment et dans d'autres matériaux. La crémaillère 3 peut par exemple être réalisée en acier inoxydable.

Ensuite le procédé de fabrication du dispositif d'éjection comprend les trois étapes essentielles décrites précédemment, permettant d'obtenir le remplissage et l'assemblage final de ces composants.

Le procédé de fabrication a été décrit dans le cadre d'un dispositif de type seringue anesthésiante dans le domaine dentaire. Plus généralement, ce procédé est adapté pour fabriquer un dispositif d'éjection destiné au domaine médical pour l'injection de produits tels que des anesthésiques dans des tissus durs ou pour le dépôt de colles, de résines ou d'amalgames. Il peut également être utilisé dans le domaine paramédical pour déposer des quantités déterminées de collagène. Il peut en outre être utilisé dans le domaine de la micromécanique et de la bijouterie pour effectuer des collages ou des microsoudures ou encore pour déposer des produits. Ainsi, le porte outil 12 présent à l'extrémité 13 du corps du dispositif peut maintenir d'autres outils qu'une aiguille 16.

Finalement, la solution atteint bien les objets recherchés et présente les avantages suivants :
- le dispositif repose sur une structure simple avec peu d'éléments, sans ressort ni axe distinct et indépendant, réalisables facilement et à bas coûts, par des procédés compatibles avec l'emploi de matériaux recyclables : le procédé de fabrication est donc économique et compatible avec un concept de dispositif jetable, ce qui peut ainsi permettre d'éliminer les problèmes de nettoyage et atteindre un niveau d'hygiène grandement supérieur ;
- le procédé permet un remplissage facile du dispositif d'éjection, qui précède une ultime phase d'assemblage qui peut être irréversible et imposer ainsi le caractère jetable du dispositif pour atteindre une hygiène garantie ;
- le remplissage simple et facile du procédé selon l'invention évite d'avoir à prévoir une étape de remplissage par un procédé industriel et permet ainsi une grande simplification du procédé de fabrication ;
- la composition du dispositif en peu d'éléments faciles à assembler permet sa distribution facile, son assemblage et remplissage final au moment du besoin d'une injection. Cette solution permet donc de commercialiser un produit intermédiaire comprenant au moins deux parties non assemblées du dispositif, un porte conteneur 2a et un corps 2b, ainsi qu'une tirette. Cela permet d'autre part à l'utilisateur final de pouvoir choisir le produit qu'il souhaite injecter, ainsi que la dose souhaitée ;
- la présence d'un levier parfaitement fixé de manière verrouillée permet d'atteindre une éjection performante.

Le dispositif selon la présente invention a été conçu sur la base du concept de dispositif jetable. Toutefois, son utilisation non jetable, en utilisant les procédés de nettoyage de l'art antérieur, ne sortirait pas pour autant du cadre de l'invention. En effet, la simplicité du procédé de fabrication permet de le rendre démontable tout aussi facilement, par exemple en modifiant la liaison entre les corps 2a et 2b pour faciliter l'échappement des bras 9. Dans une telle solution, son nettoyage serait grandement simplifié du fait de la simplification de la structure du dispositif.

## Revendications

1. Procédé de fabrication d'un dispositif d'éjection d'un produit liquide ou pâteux (11), **caractérisé en ce qu'**il comprend:
- au moins trois étapes de moulage par injection d'un matériau plastique pour former trois parties distinctes du dispositif ; un levier (1) intégrant un cliquet (4), un porte conteneur (2a) et un corps arrière (2b),
- une étape par laquelle une tirette (18) est insérée dans une ouverture arrière (17) du porte conteneur (2a) et reliée au piston (19) délimitant un volume (10) à injecter,
- une étape d'aspiration du produit (11) à éjecter au sein du volume (10) du porte conteneur (2a) du dispositif d'éjection à l'aide de la tirette (18),
- une étape de démontage de la tirette (18) du porte conteneur (2a),
- une étape intermédiaire d'assemblage du levier (1) sur le corps arrière (2b), suivie de l'insertion d'une crémaillère (3) par une ouverture arrière (7), et,
- une étape d'assemblage irréversible du corps arrière (2b) sur le porte conteneur (2a) pour former le dispositif d'éjection prêt à l'emploi.

2. Procédé de fabrication d'un dispositif d'éjection d'un produit liquide ou pâteux selon la revendication précédente, caractérisé en ce la liaison entre la tirette (18) et le piston (19) du porte conteneur (2a) se fait par un vissage de l'extrémité (40) de la tirette (18) dans le piston (19).

3. Procédé de fabrication d'un dispositif d'éjection d'un produit liquide ou pâteux selon l'une des revendications précédentes, **caractérisé en ce qu'**il comprend une étape préalable de fixation d'un outil à l'extrémité avant (13) du porte conteneur (2a).

4. Procédé de fabrication d'un dispositif d'éjection d'un produit liquide ou pâteux selon la revendication précédente, **caractérisé en ce que** l'extrémité antérieure (13) du porte conteneur (2a) présente une forme globalement inclinée par rapport au corps (2a) mais présentant une surface ayant au moins une génératrice sensiblement parallèle à l'axe de ce corps (2a) afin de permettre une insertion droite d'un outil de type aiguille (16) dans cette direction avant sa flexion et fixation en position de travail inclinée.

5. Procédé de fabrication d'un dispositif d'éjection d'un produit liquide ou pâteux selon l'une des revendications 1 à 3, **caractérisé en ce qu'**il comprend une étape préalable d'assemblage avec le porte conteneur (2a) d'une seringue (10') présentant un corps cylindrique allongé délimité dans sa partie arrière par un piston (19') mobile en translation longitudinale, et comprenant dans sa partie avant une aiguille (16') maintenue par un embout (12').

6. Procédé de fabrication d'un dispositif d'éjection d'un produit liquide ou pâteux selon l'une des revendications précédentes, **caractérisé en ce que** le levier (1) comprend une partie supérieure horizontale s'étendant vers l'avant, destinée à être manipulée, et une extrémité inférieure en forme d'axe (5) qui est positionnée dans un emplacement (6) correspondant du corps qu'il arrière (2b).

7. Procédé de fabrication d'un dispositif d'éjection d'un produit liquide ou pâteux selon l'une des revendications précédentes, **caractérisé en ce que** l'étape d'assemblage du corps arrière (2b) avec le porte conteneur (2a) est réalisé par clipsage d'au moins deux lames latérales (9) d'un premier élément, s'étendant longitudinalement et présentant une protubérance (31) orientée vers l'extérieur, destinées à coopérer avec des ouvertures correspondantes (8) sur le second élément.

8. Procédé de fabrication d'un dispositif d'éjection d'un produit liquide ou pâteux selon la revendication précédente, **caractérisé en ce qu'**il comprend une quatrième étape d' injection d'un matériaux plastique pour former une crémaillère (3).

9. Procédé de fabrication d'un dispositif d'éjection d'un produit liquide ou pâteux selon les revendications 8 et 9, **caractérisé en ce que** l'étape d'assemblage de la crémaillère (3) au sein du dispositif est apte à permettre un mouvement dans certaine direction pour induire l'éjection du produit et à rendre impossible le mouvement dans la direction opposée pour empêcher un second remplissage du dispositif.

10. Procédé de fabrication d'un dispositif d'éjection d'un produit liquide ou pâteux selon l'une des revendications précédentes, **caractérisé en ce que** l'assemblage du corps arrière (2b) sur le porte conteneur (2a) comprend une liaison entre un moyen d'éjection (1, 3, 4) du corps arrière (2b) et un piston (19 ; 19') du porte conteneur (2a) de sorte de pouvoir éjecter le produit (11) du porte conteneur (2a) par une action sur ce moyen d'éjection du corps arrière.

11. Kit pour la mise en oeuvre du procédé de fabrication selon l'une des revendications précédentes, **caractérisé en ce qu'**il comprend un porte conteneur (2a), un corps arrière (2b), un piston (19, 19') du porte conteneur (2a) pour fermer le volume (10) à remplir, un levier (1) intégrant un cliquet (4), une tirette (18) et une crémaillère (3).

12. Kit de fabrication d'un dispositif d'éjection d'un produit liquide ou pâteux selon la revendication précédente, **caractérisé en ce que** la tirette (18) est distincte du porte conteneur (2a) et présente un organe de liaison (40) pour sa liaison directe ou indirecte avec ledit piston (19 ; 19') du porte conteneur (2a).

13. Kit de fabrication d'un dispositif d'éjection d'un produit liquide ou pâteux selon l'une des revendications 12 à 13, **caractérisé en ce qu'**il comprend de plus un porte outil (12) et un outil de type aiguille (16), aptes à une liaison avec le porte conteneur (2a).

## Patentansprüche

1. Herstellungsverfahren einer Ausstoßvorrichtung eines flüssigen oder pastösen Produkts (11), **dadurch gekennzeichnet, dass** es umfasst:
- mindestens drei Formungsschritte durch Spritzguss eines Kunststoffmaterials, um drei unterschiedliche Teile der Vorrichtung zu bilden; einen Hebel (1) mit einer Sperrklinke (4), einen Behälterhalter (2a) und einen hinteren Körper (2b),
- einen Schritt, bei dem ein Zug (18) in eine hintere Öffnung (17) des Behälterhalters (2a) eingesetzt und mit dem Kolben (19) verbunden wird, der ein einzuspritzendes Volumen (10) begrenzt,
- einen Ansaugschritt des auszustoßenden Produkts (11) in das Volumen (10) des Behälterhalters (2a) der Ausstoßvorrichtung mit Hilfe des Zugs (18),
- einen Schritt der Demontage des Zugs (18) vom Behälterhalter (2a),
- einen Zwischenschritt des Verbindens des Hebels (1) mit dem hinteren Körper (2b), gefolgt vom Einsetzen einer Zahnstange (3) durch eine hintere Öffnung (7), und
- einen Schritt des unlöslichen Verbindens des hinteren Körpers (2b) mit dem Behälterhalter (2a), um die gebrauchsfertige Ausstoßvorrichtung zu bilden.

2. Herstellungsverfahren einer Ausstoßvorrichtung eines flüssigen oder pastösen Produkts nach vorangehendem Anspruch, **dadurch gekennzeichnet, dass** die Verbindung zwischen dem Zug (18) und dem Kolben (19) des Behälterhalters (2a) durch Schrauben des Endes (40) des Zugs (18) in den Kolben (19) erfolgt.

3. Herstellungsverfahren einer Ausstoßvorrichtung eines flüssigen oder pastösen Produkts nach einem der vorangehenden Ansprüche, **dadurch gekennzeichnet, dass** es einen vorherigen Schritt des Befestigens eines Werkzeugs am vorderen Ende (13) des Behälterhalters (2a) umfasst.

4. Herstellungsverfahren einer Ausstoßvorrichtung eines flüssigen oder pastösen Produkts nach vorangehendem Anspruch, **dadurch gekennzeichnet, dass** das vordere Ende (13) des Behälterhalters (2a) im Verhältnis zum Körper (2a) eine allgemein geneigte Form aufweist, aber eine Oberfläche hat, die mindestens eine zur Achse dieses Körpers (2a) etwa parallele Mantellinie hat, um ein gerades Einsetzen eines Werkzeugs vom Typ Nadel (16) in dieser Richtung vor seiner Biegung und Befestigung in geneigter Arbeitsposition zu erlauben.

5. Herstellungsverfahren einer Ausstoßvorrichtung eines flüssigen oder pastösen Produkts nach einem der Ansprüche 1 bis 3, **dadurch gekennzeichnet, dass** es einen vorherigen Schritt des Verbindens mit dem Behälterhalter (2a) einer Spritze (10') umfasst, aufweisend eine länglichen zylindrischen Körper, der in seinem hinteren Abschnitt von einem in Längsverschiebung bewegbaren Kolben (19') begrenzt ist und in seinem vorderen Abschnitt eine Nadel (16') umfasst, die von einem Ansatz (12') gehalten wird.

6. Herstellungsverfahren einer Ausstoßvorrichtung eines flüssigen oder pastösen Produkts nach einem der vorangehenden Ansprüche, **dadurch gekennzeichnet, dass** der Hebel (1) einen oberen horizontalen Abschnitt umfasst, der sich nach vorn erstreckt, der bestimmt ist, bedient zu werden, und ein unteres Ende in Form einer Achse (5), die an einer Stelle (6) positioniert ist, die dem hinteren Körper (2b) entspricht.

7. Herstellungsverfahren einer Ausstoßvorrichtung eines flüssigen oder pastösen Produkts nach einem der vorangehenden Ansprüche, **dadurch gekennzeichnet, dass** der Schritt des Verbindens des hinteren Körpers (2b) mit dem Behälterhalter (2a) durch Rasten von mindestens zwei seitlichen Klingen (9) eines ersten Elements, die sich länglich erstrecken und einen nach außen gerichteten Vorsprung (31) aufweisen, erfolgt, die bestimmt sind, mit entsprechenden Öffnungen (8) auf dem zweiten Element zusammenzuwirken.

8. Herstellungsverfahren einer Ausstoßvorrichtung eines flüssigen oder pastösen Produkts nach vorangehendem Anspruch, **dadurch gekennzeichnet, dass** es einen vierten Schritt des Einspritzens eines Kunststoffmaterials umfasst, um eine Zahnstange (3) zu bilden.

9. Herstellungsverfahren einer Ausstoßvorrichtung eines flüssigen oder pastösen Produkts nach den Ansprüchen 8 und 9, **dadurch gekennzeichnet, dass** der Schritt des Verbindens der Zahnstange (3) in der Vorrichtung imstande ist, eine Bewegung in eine bestimmte Richtung zu erlauben, um das Ausstoßen des Produkts zu induzieren und um die Bewegung in die entgegengesetzte Richtung unmöglich zu machen, um ein zweites Befüllen der Vorrichtung zu verhindern.

10. Herstellungsverfahren einer Ausstoßvorrichtung eines flüssigen oder pastösen Produkts nach einem der vorangehenden Ansprüche, **dadurch gekennzeichnet, dass** das Verbinden des hinteren Körpers (2b) mit dem Behälterhalter (2a) eine Verbindung zwischen einem Ausstoßmittel (1, 3, 4) des hinteren Körpers (2b) und einem Kolben (19; 19') des Behälterhalters (2a) derart umfasst, dass das Produkt (11) aus dem Behälterhalter (2a) mittels einer Aktion auf dieses Ausstoßmittel des hinteren Körpers ausgestoßen werden kann.

11. Set für die Umsetzung des Herstellungsverfahrens nach einem der vorangehenden Ansprüche, **dadurch gekennzeichnet, dass** es einen Behälterhalter (2a), einen hinteren Körper (2b), einen Kolben (19, 19') des Behälterhalters (2a) zum Verschließen des zu füllenden Volumens (10), einen Hebel (1) mit einer Sperrklinke (4), einen Zug (18) und eine Zahnstange (3) umfasst.

12. Set für die Herstellung einer Ausstoßvorrichtung eines flüssigen oder pastösen Produkts nach vorangehendem Anspruch, **dadurch gekennzeichnet, dass** sich der Zug (18) vom Behälterhalter (2a) unterscheidet und ein Verbindungsorgan (40) für seine direkte oder indirekte Verbindung mit dem Kolben (19; 19') des Behälterhalters (2a) aufweist.

13. Set für die Herstellung einer Ausstoßvorrichtung eines flüssigen oder pastösen Produkts nach einem der Ansprüche 12 bis 13, **dadurch gekennzeichnet, dass** es zusätzlich einen Werkzeughalter (12) und ein Werkzeug vom Typ Nadel (16) umfasst, die zu einer Verbindung mit dem Behälterhalter (2a) befähigt sind.

## Claims

1. A method for manufacturing a device for ejecting a liquid or pasty product (11), **characterized in that** it comprises:
- at least three steps for injection molding a plastic material to form three separate parts of the device; a lever (1) integrating a click (4), a container holder (2a) and a rear body (2b),
- a step by which a pull tab (18) is inserted into a rear opening (17) of the container holder (2a) and connected to the piston (19) defining a volume (10) to be injected,
- a step for aspirating the product (11) to be injected within the volume (10) of the container holder (2a) of the ejection device using the pull tab (18),
- a step for disassembling the pull tab (18) from the container holder (2a),
- an intermediate step for assembling the lever (1) on the rear body (2b), followed by the insertion of a rack (3) through a rear opening (7), and
- a step for irreversibly assembling the rear body (2b) on the container holder (2a) to form the ready-to-use ejection device.

2. The method for manufacturing a device for ejecting a liquid or pasty product according to the preceding claim, **characterized in that** the connection between the pull tab (18) and the piston (19) of the container holder (2a) is done by screwing the end (40) of the pull tab (18) into the piston (19).

3. The method for manufacturing a device for ejecting a liquid or pasty product according to one of the preceding claims, **characterized in that** it comprises a prior step for fastening a tool to the front end (13) of the container holder (2a).

4. The method for manufacturing a device for ejecting a liquid or pasty product according to the preceding claim, **characterized in that** the front end (13) of the container holder (2a) has a globally inclined shape relative to the body (2a), but having a surface having at least one generatrix substantially parallel to the axis of this body (2a) in order to allow a straight insertion of a tool of the needle type (16) in this direction before flexion and fastening in the end inclined working position thereof.

5. The method for manufacturing a device for injecting a liquid or pasty product according to one of claims 1 to 3, **characterized in that** it comprises a step for prior assembly with the container holder (2a) of a syringe (10') having an elongated cylindrical body defined in its rear part by a longitudinally translatable piston (19'), and comprising a needle (16') in its front part that is maintained by an end-piece (12').

6. The method for manufacturing a device for ejecting a liquid or pasty product according to one of the preceding claims, **characterized in that** the lever (1) comprises a horizontal upper part extending forward, designed to be manipulated, and a lower end in the form of an axle (5) that is positioned in a corresponding location (6) of the [rear body] (2b).

7. The method for manufacturing a device for ejecting a liquid or pasty product according to one of the preceding claims, **characterized in that** the step for assembling the rear body (2b) with the container holder (2a) is done by clipping at least two lateral blades (9) of a first element, extending longitudinally and having a protuberance (31) oriented toward the outside, intended to cooperate with corresponding openings (8) on the second element.

8. The method for manufacturing a device for ejecting a liquid or pasty product according to the preceding claim, **characterized in that** it comprises a fourth step for injecting a plastic material to form a rack (3).

9. The method for manufacturing a device for ejecting a liquid or pasty product according to claims 8 or 9, **characterized in that** the step for assembling the rack (3) within the device is able to allow a movement in a certain direction to cause the ejection of the product and make movement in the opposite direction impossible to prevent a second filling of the device.

10. The method for manufacturing a device for ejecting a liquid or pasty product according to one of the preceding claims, **characterized in that** the assembly of the rear body (2b) on the container holder (2a) comprises a connection between an ejection means (1, 3, 4) of the rear body (2b) and a piston (19; 19') of the container holder (2a) so as to be able to eject the product (11) from the container holder (2a) by an action on this ejection means of the rear body.

11. A kit for carrying out the manufacturing method according to one of the preceding claims, **characterized in that** it comprises a container holder (2a), a rear body (2b), a piston (19, 19') of the container holder (2a) to close the volume (10) to be filled, a lever (1) incorporating a click (4), a pull tab (18) and a rack (3).

12. The kit for manufacturing a device for ejecting a liquid or pasty product according to the preceding claim, **characterized in that** the pull tab (18) is separate from the container holder (2a) and has a connecting member (40) for its direct or indirect connection with said piston (19; 19') of the container holder (2a).

13. The kit for manufacturing a device for ejecting a liquid or pasty product according to one of claims 12 to 13, **characterized in that** it also comprises a tool holder (12) and a tool of the needle type (16), suitable for connection with the container holder (2a).
